(19) Europäisches Patentamt
European Patent Office
Office européen des brevets

(11) **EP 4 506 335 A1**

(12) **EUROPEAN PATENT APPLICATION**
published in accordance with Art. 153(4) EPC

(43) Date of publication:
**12.02.2025 Bulletin 2025/07**

(21) Application number: **23780882.9**

(22) Date of filing: **30.03.2023**

(51) International Patent Classification (IPC):
**C07C 319/12** (2006.01)      **C07C 321/04** (2006.01)
**C07B 61/00** (2006.01)        **C08G 18/38** (2006.01)
**G02B 1/04** (2006.01)

(52) Cooperative Patent Classification (CPC):
**C07B 61/00; C07C 319/12; C07C 321/04;
C08G 18/38; G02B 1/04**

(86) International application number:
**PCT/JP2023/013187**

(87) International publication number:
**WO 2023/190874 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.03.2022  JP 2022056057**

(71) Applicant: **HOYA LENS THAILAND LTD.
Pathumthani 12130 (TH)**

(72) Inventor: **KOUSAKA, Masahisa
Tokyo 1608347 (JP)**

(74) Representative: **Kraus & Lederer PartGmbB
Thomas-Wimmer-Ring 15
80539 München (DE)**

(54) **METHOD FOR PRODUCING PENTAERYTHRITOL MERCAPTOCARBOXYLIC ESTER, POLYMERIZABLE COMPOSITION, RESIN, OPTICAL MATERIAL, AND SPECTACLE LENS**

(57) There are provided a method of producing pentaerythritol mercaptocarboxylate including subjecting a mercaptocarboxylic acid and pentaerythritol to a dehydration reaction under solvent-free conditions, a polymerizable composition containing the pentaerythritol mercaptocarboxylate, a resin which is a cured product of the polymerizable composition, an optical material containing the resin, and a spectacle lens containing the resin.

**EP 4 506 335 A1**

## Description

[Technical Field]

The present disclosure relates to a method of producing pentaerythritol mercaptocarboxylate, a polymerizable composition, a resin, an optical material, and a spectacle lens.

[Background Art]

Plastic spectacle lenses are widely known in the related art. A polythiourethane resin is used as a material for spectacle lenses (for example, PTL 1). Since the polythiourethane resin is a material having an appropriate refractive index, it allows a spectacle lens to have an appropriate thickness, and easily secures processability and impact resistance, and is widely used. Pentaerythritol mercaptocarboxylate is used as a monomer for a polythiourethane resin (PTL 2).

[Citation List]

[Patent Literature]

[PTL 1] Japanese Patent Application Publication No. 2004-002820
[PTL 2] WO 2016/208707

[Summary of Invention]

[Technical Problem]

Pentaerythritol mercaptocarboxylate can be obtained by subjecting a mercaptocarboxylic acid and pentaerythritol to a dehydration reaction. The dehydration reaction is a reversible equilibrium reaction, and the conversion rate can be increased by performing the reaction while discharging water from the system. Therefore, toluene, which forms an azeotrope with water, is used, refluxing is performed using a Dean-Stark trap or the like, and water is discharged from the reaction system. On the other hand, there are problems that the use of the toluene solvent causes a high purification load and a product to become colored.
One embodiment of the present disclosure provides a method of producing pentaerythritol mercaptocarboxylate with less purification load and less coloration, a polymerizable composition containing the pentaerythritol mercaptocarboxylate, a resin which is a cured product of the polymerizable composition, an optical material containing the resin, and a spectacle lens containing the resin.

[Solution to Problem]

The inventors found that the above problems can be solved by subjecting a mercaptocarboxylic acid and pentaerythritol to a dehydration reaction under solvent-free conditions.
One embodiment according to the present disclosure relates to a method of producing pentaerythritol mercaptocarboxylate including subjecting a mercaptocarboxylic acid and pentaerythritol to a dehydration reaction under solvent-free conditions.

[Advantageous Effects of Invention]

According to one embodiment of the present disclosure, it is possible to provide a method of producing pentaerythritol mercaptocarboxylate with less purification load and less coloration, a polymerizable composition containing the pentaerythritol mercaptocarboxylate, a resin which is a cured product of the polymerizable composition, an optical material containing the resin, and a spectacle lens containing the resin.

[Description of Embodiments]

Hereinafter, an embodiment (hereinafter referred to as "the present embodiment") of the present disclosure will be described in detail, but the present invention is not limited thereto, and can be variously modified without departing from the scope and spirit thereof. Here, in this specification, for example, the expression of a numerical value range of "1 to 100" includes both the lower limit value "1" and the upper limit value "100." In addition, the same applies to the expression of other numerical value ranges.

[Method of producing pentaerythritol mercaptocarboxylate]

One embodiment according to the present disclosure relates to a method of producing pentaerythritol mercaptocarboxylate including subjecting a mercaptocarboxylic acid and pentaerythritol to a dehydration reaction under solvent-free conditions. According to one embodiment of the present disclosure, it is possible to provide a method of producing pentaerythritol mercaptocarboxylate with less purification load and less coloration, a polymerizable composition containing the pentaerythritol mercaptocarboxylate, a resin which is a cured product of the polymerizable composition, an optical material containing the resin, and a spectacle lens containing the resin.

The method of producing pentaerythritol mercaptocarboxylate according to the present embodiment includes, for example,

subjecting a mercaptocarboxylic acid and pentaerythritol to a dehydration reaction under solvent-free conditions (hereinafter referred to as "reaction process"), and
washing the pentaerythritol mercaptocarboxylate obtained by the dehydration reaction (hereinafter referred to as "washing process").

The processes will be described below in detail.

[Reaction process]

In the reaction process, a mercaptocarboxylic acid and pentaerythritol are subjected to a dehydration reaction under solvent-free conditions. When the reaction is performed under solvent-free conditions in the reaction process, since it is unnecessary to distill off the solvent, it is possible to reduce the load of subsequent purification.

Here, "under solvent-free conditions" means conditions in which no organic solvent such as toluene is used, and specifically means conditions in which the amount of the organic solvent is 20 mass% or less based on a total mass of mercaptocarboxylic acid and pentaerythritol. The amount of the organic solvent is preferably 10 mass% or less, more preferably 5 mass% or less, and still more preferably 1 mass% or less based on a total mass of mercaptocarboxylic acid and pentaerythritol.

(Mercaptocarboxylic acid)

Mercaptocarboxylic acid preferably has 2 to 4 carbon atoms and more preferably has 2 to 3 carbon atoms. Mercaptocarboxylic acid is preferably 2-mercaptoacetic acid or 3-mercaptopropionic acid. In addition, an ester bond is formed by a dehydration reaction which is a reaction between a mercaptocarboxylic acid and pentaerythritol, and pentaerythritol mercaptocarboxylate is obtained.

The pentaerythritol preferably has a purity of 90 mass% or more, more preferably a purity of 95 mass% or more, and still more preferably has a purity of 98 mass% or more.

The pentaerythritol may contain more than 1.0 mass% of sodium. Even when it contains such a high concentration of sodium, pentaerythritol mercaptocarboxylate having a low Hazen color scale value (APHA) is obtained.

The molar ratio between mercaptocarboxylic acid and pentaerythritol (mercaptocarboxylic acid/pentaerythritol) is preferably 3.5 to 6.0, more preferably 4.0 to 5.0, and still more preferably 4.1 to 5.5.

In the reaction process, the dehydration reaction is preferably performed in the presence of a sulfonic acid compound. The sulfonic acid compound acts as an acid catalyst and promotes the dehydration reaction.

Examples of sulfonic acid compounds include aromatic sulfonic acids such as toluenesulfonic acid, and alkylsulfonic acids. Among these, alkylsulfonic acids are preferable in order to reduce coloration. Examples of alkylsulfonic acids include methanesulfonic acid, ethanesulfonic acid, 1-propanesulfonic acid, and trifluoromethanesulfonic acid. Among these, methanesulfonic acid is preferable in order to reduce coloration.

The amount of the sulfonic acid compound is preferably 0.1 to 10 mass%, more preferably 0.2 to 5 mass%, and still more preferably 0.3 to 3 mass% based on a total amount of mercaptocarboxylic acid and pentaerythritol.

The reaction process is preferably performed under a reduced pressure while removing water from the system. When water is removed from the system, the equilibrium of the ester reaction can be shifted toward the product and the yield can be improved.

The reaction process is preferably performed at a temperature of 90 to 100°C in order to reduce coloration. The temperature during the reaction process is preferably 90 to 98°C, more preferably 92 to 97°C, and still more preferably 93 to 96°C.

[Washing process]

In the washing process, the pentaerythritol mercaptocarboxylate obtained in the reaction process is washed. The washing is performed, for example, by washing the mixture obtained in the reaction process with water. Thereby, the catalyst such as a sulfonic acid compound can be removed.

[Pentaerythritol mercaptocarboxylate]

Pentaerythritol mercaptocarboxylate is obtained by the production method according to the present embodiment.

The Hazen color scale value (APHA) of the obtained pentaerythritol mercaptocarboxylate is preferably 15 or less, more preferably 10 or less, still more preferably 7 or less, and yet more preferably 6 or less.

The measured value (g/eq) of the SH value of the obtained pentaerythritol mercaptocarboxylate/theoretical value (g/eq) of the SH value of the pentaerythritol mercaptocarboxylate (measured value/theoretical value) is preferably 1.10 or less, more preferably 1.08 or less, and still more preferably 1.06 or less. The measured value of the SH value is obtained by the measurement method described in examples. The theoretical value of the SH value is obtained by the following Formula (A) .

[theoretical value of SH value]=[molecular weight]/[number of SH groups in one molecule] ...                    (A)

[Polymerizable composition]

The polymerizable composition according to the present embodiment contains the pentaerythritol mercaptocarboxylate obtained by the production method according to the present embodiment and a polyiso(thio)cyanate. The pentaerythritol mercaptocarboxylate is polymerized with reacting with the polyiso(thio)cyanate to obtain a cured product.

(Polyiso(thio)cyanate)

Examples of polyiso(thio)cyanates include polyisocyanate compounds having an aromatic ring, alicyclic polyisocyanate compounds, and linear or branched aliphatic polyisocyanate compounds.

Examples of polyisocyanate compounds having an aromatic ring include diisocyanatobenzene, 2,4-tolylene diisocyanate, 2,6-tolylene diisocyanate, ethyl phenylene diisocyanate, isopropyl phenylene diisocyanate, dimethyl phenylene diisocyanate, diethyl phenylene diisocyanate, diisopropyl phenylene diisocyanate, trimethylbenzene triisocyanate, benzene triisocyanate, biphenyl diisocyanate, toluidine diisocyanate, 4,4'-diphenylmethane diisocyanate, 4,4'-methylenebis(2-methylphenylisocyanate), bibenzyl-4,4'-diisocyanate, bis(isocyanatophenyl)ethylene, 1,3-bis(isocyanatomethyl)benzene, 1,4-bis(isocyanatomethyl)benzene, 1,3-bis(isocyanatoethyl)benzene, bis(isocyanatopropyl)ben-zene, $\alpha,\alpha,\alpha',\alpha'$-tetramethylxylylene diisocyanate, bis(isocyanatobutyl)benzene, bis(isocyanatomethyl)naphthalene, bis(isocyanatomethylphenyl)ether, 2-isocyanatophenyl-4-isocyanatophenyl sulfide, bis(4-isocyanatophenyl)sulfide, bis(4-isocyanatomethylphenyl)sulfide, bis(4-isocyanatophenyl)disulfide, bis(2-methyl-5-isocyanatophenyl)disulfide, bis(3-methyl-5-isocyanatophenyl)disulfide, bis(3-methyl-6-isocyanatophenyl)disulfide, bis(4-methyl-5-isocyanatophenyl)disulfide, bis(3-methyloxy-4-isocyanatophenyl)disulfide, and bis(4-methyloxy-3-isocyanatophenyl)disulfide.

Examples of alicyclic polyisocyanate compounds include 1,3-diisocyanatocyclohexane, isophorone diisocyanate, 1,3-bis(isocyanatomethyl)cyclohexane, 1,4-bis(isocyanatomethyl)cyclohexane, dicyclohexylmethane-4,4'-diisocyanate, 2,5-bis(isocyanatomethyl)-bicyclo[2.2.1]heptane, 2,6-bis(isocyanatomethyl)-bicyclo[2.2.1]heptane, 2,5-diisocyanato-1,4-dithiane, 2,5-bis(isocyanatomethyl)-1,4-dithiane, 4,5-diisocyanato-1,3-dithiolane, 4,5-bis(isocyanatomethyl)-1,3-dithiolane, and 4,5-bis(isocyanatomethyl)-2-methyl-1,3-dithiolane.

Examples of linear or branched aliphatic polyisocyanate compounds include hexamethylene diisocyanate, 2,2-dimethylpentane diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecane triisocyanate, 1,3,6-hexamethylenetriisocyanate, 1,8-diisocyanato-4-isocyanatomethyloctane, bis(isocyanatoethyl)carbonate, bis(isocyanatoethyl)ether, lysine diisocyanatomethyl ester, lysine triisocyanate, bis(isocyanatomethyl)sulfide, bis(isocyanatoethyl)sulfide, bis(isocyanatopropyl) sulfide, bis(isocyanatohexyl)sulfide, bis(isocyanatomethyl)sulfone, bis(isocyanatomethyl)disulfide, bis(isocyanatoethyl) disulfide, bis(isocyanatopropyl)disulfide, bis(isocyanatomethylthio)methane, bis(isocyanatoethylthio)methane, bis(isocyanatomethylthio)ethane, bis(isocyanatoethylthio)ethane, 1,5-diisocyanato-2-isocyanatomethyl-3-pentane, 1,2,3-tris(isocyanatomethylthio)propane, 1,2,3-tris(isocyanatoethylthio)propane, 3,5-dithia-1,2,6,7-heptanetetraisocyanate, 2,6-diisocyanatomethyl-3,5-dithia-1,7-heptane diisocyanate, 2,5-diisocyanatomethylthiophene, 4-isocyanatoethylthio-2,6-dithia-1,8-octanediisocyanate, 1,2-diisothiocyanatoethane, and 1,6-diisothiocyanatohexane.

These may be used alone or two or more thereof may be used.

The polyiso(thio)cyanate preferably contains at least one selected from the group consisting of bis(isocyanatomethyl)bicyclo[2.2.1]heptane, bis(isocyanatomethyl)cyclohexane, bis(isocyanatomethyl)benzene, tolylene diisocyanate, diphenylmethane diisocyanate, dicyclohexylmethane diisocyanate, hexamethylene diisocyanate, and pentamethylene diisocyanate (hereinafter referred to as "preferable isocyanate compound"), and more preferably contains at least one selected from the group consisting of bis(isocyanatomethyl)benzene, tolylene diisocyanate, and diphenylmethane diisocyanate.

Bis(isocyanatomethyl)bicyclo[2.2.1]heptane includes, for example, one or more selected from the group consisting of 2,5-bis(isocyanatomethyl)bicyclo[2.2.1]heptane and 2,6-bis(isocyanatomethyl)bicyclo[2.2.1]heptane, and is preferably a mixture of 2,5-bis(isocyanatomethyl)bicyclo[2.2.1]heptane and 2,6-bis(isocyanatomethyl)bicyclo[2.2.1]heptane.

Examples of bis(isocyanatomethyl)cyclohexane include 1,3-bis(isocyanatomethyl)cyclohexane and 1,4-bis(isocyanatomethyl)cyclohexane. Among these, 1,3-bis(isocyanatomethyl)cyclohexane is preferable.

Examples of bis(isocyanatomethyl)benzene include 1,3-bis(isocyanatomethyl)benzene and 1,4-bis(isocyanatomethyl)benzene. Among these, 1,3-bis(isocyanatomethyl)benzene is preferable.

Examples of tolylene diisocyanates include 2,4-tolylene diisocyanate and 2,6-tolylene diisocyanate. Among these, 2,4-tolylene diisocyanate is preferable.

Examples of diphenylmethane diisocyanates include 4,4'-diphenylmethane diisocyanate.

Examples of dicyclohexylmethane diisocyanates include dicyclohexylmethane-4,4'-diisocyanate.

The polyiso(thio)cyanate compound preferably includes at least one selected from among bis(isocyanatomethyl)-bicyclo[2.2.1]heptane, 1,3-bis(isocyanatomethyl)cyclohexane, 1,4-bis(isocyanatomethyl)cyclohexane, bis(isocyanatomethyl)benzene, isophorone diisocyanate, pentamethylene diisocyanate, and hexamethylene diisocyanate, and among these, bis(isocyanatomethyl)bicyclo[2.2.1]heptane, 1,3-bis(isocyanatomethyl)cyclohexane, or bis(isocyanatomethyl)benzene is preferable.

In the polyiso(thio)cyanate, the content of the above "preferable isocyanate compound" is preferably 80 mass% or more, more preferably 90 mass% or more, and still more preferably 95 mass% or more and 100 mass% or less.

The equivalent ratio between the mercapto groups of the pentaerythritol mercaptocarboxylate and the isocyanate groups of the polyiso(thio)cyanate (mercapto groups/isocyanate groups) is preferably 40/60 or more, more preferably 43/57 or more, still more preferably 45/55 or more, and preferably 60/40 or less, more preferably 55/45 or less, and still more preferably 53/47 or less.

A polymerization catalyst may be used for curing the isocyanate component and the thiol component.

Examples of polymerization catalysts include tin compounds and nitrogen-containing compounds.

Examples of tin compounds include alkyltin compounds and alkyltin halide compounds.

Examples of alkyltin compounds include dibutyltin diacetate and dibutyltin dilaurate.

Examples of alkyltin halide compounds include dibutyltin dichloride, dimethyltin dichloride, monomethyltin trichloride, trimethyltin chloride, tributyltin chloride, tributyltin fluoride, and dimethyltin dibromide.

Among these, dibutyltin diacetate, dibutyltin dilaurate, dibutyltin dichloride, and dimethyltin dichloride are preferable, and dimethyltin dichloride is more preferable.

Examples of nitrogen-containing compounds include tertiary amines, quaternary ammonium salts, imidazole compounds, and pyrazole compounds. Tertiary amines are preferably hindered amines.

Examples of tertiary amines include triethylamine, tri-n-propylamine, triisopropylamine, tri-n-butylamine, triisobutylamine, N,N-dimethylbenzylamine, N-methylmorpholine, N,N-dimethylcyclohexylamine, pentamethyldiethylenetriamine, bis(2-dimethylaminoethyl) ether, N-methylmorpholine, N,N'-dimethylpiperazine, N,N,N',N'-tetramethylethylenediamine, and 1,4-diazabicyclo[2.2.2]octane(DABCO).

Examples of hindered amines include 1,2,2,6,6-pentamethyl-4-piperidinol, 1,2,2,6,6-pentamethyl-4-hydroxyethyl-4-piperidinol, methyl-1,2,2,6,6-pentamethyl-4-piperidyl sebacate, a mixture of methyl-1,2,2,6,6-pentamethyl-4-piperidyl sebacate and bis(1,2,2,6,6-pentamethyl-4-piperidyl)sebacate, bis(1,2,2,6,6-pentamethyl-4-piperidyl)sebacate, bis(2,2,6,6-tetramethyl-1-(octyloxy)-4-piperidyl)sebacate, bis(1,2,2,6,6-pentamethyl-4-piperidyl)[[3,5-bis(1,1-dimethylethyl)-4-hydroxyphenyl]methyl]butylmalonate, and tetrakis(1,2,2,6,6-pentamethyl-4-piperidyl)butane-1,2,3,4-tetracarboxylate.

Examples of quaternary ammonium salts include tetraethylammonium hydroxide.

Examples of imidazole compounds include imidazole, 1-methyl-2-mercapto-1H-imidazole, 1,2-dimethylimidazole, benzylmethylimidazole, and 2-ethyl-4-imidazole.

Examples of pyrazole compounds include pyrazole and 3,5-dimethylpyrazole.

Among these, tertiary amines such as hindered amines, imidazole compounds, and pyrazole compounds are preferable, imidazole compounds are more preferable, and 1-methyl-2-mercapto-1H-imidazole is still more preferable.

The amount of the polymerization catalyst used is preferably 0.001 to 2 parts by mass, more preferably 0.005 to 1 part by mass, and still more preferably 0.007 to 0.5 parts by mass based on a total amount of 100 parts by mass of the isocyanate component and the thiol component.

[Resin]

The resin according to the present embodiment is a cured product of the polymerizable composition according to the above embodiment. The cured product is obtained by polymerizing the components in the polymerizable composition.

Polymerization conditions can be appropriately set depending on the polymerizable composition. The polymerization start temperature is preferably 0°C or higher, more preferably 10°C or higher, and preferably 50°C or lower, and more preferably 40°C or lower. Preferably, the temperature is raised from the polymerization start temperature and curing by heating is then performed. For example, the maximum heating temperature is generally 110°C or higher and 130°C or lower.

Resins are used in various optical materials such as spectacle lenses, camera lenses, prisms, optical fibers, substrates used therefor, substrates for recording media used for optical disks, magnetic disks or the like, and optical filters attached to computer displays. Among these optical materials, resins are preferably used as spectacle lenses.

[Spectacle lens]

The spectacle lens according to the present embodiment contains the resin according to the present embodiment.

In addition, the spectacle lens according to the present embodiment preferably includes a lens substrate containing the resin according to the present embodiment.

(Lens substrate)

The lens substrate contains the resin according to the present embodiment in an amount of preferably 90 mass% or more, more preferably 95 mass% or more, and still more preferably 99 mass% or more.

The lens substrate may contain other additives such as a mold releasing agent, a coloring agent, an antioxidant, an anti-coloring agent, and a fluorescent brightening agent. These may be used alone or two or more thereof may be used.

The lens substrate may be either a finished lens or a semi-finished lens. The surface shape of the lens substrate is not particularly limited, and may be flat, convex, concave or the like. The lens substrate may be used for any application such as a single focal lens, a multifocal lens, and a progressive power lens. For example, as an example, for a progressive power lens, generally, a near portion region (near portion) and a progressive portion region (intermediate region) are included in the above lower region, and a distance portion region (distance portion) is included in an upper region. As the lens substrate, a colorless lens substrate is generally used, but a lens substrate that is colored in a range in which the transparency is not impaired can also be used.

The refractive index ne of the lens substrate is preferably 1.60 or more. The upper limit of the refractive index ne of the lens substrate is not particularly limited, and may be, for example, 1.80 or less.

The spectacle lens according to the present embodiment may include at least one layer selected from the group consisting of a hard coat layer, a foundation layer, and an antireflection layer.

[Method of producing spectacle lens]

The method of producing a spectacle lens according to the present embodiment includes curing, in a molding die, a polymerizable composition containing a thiol component including the compound (1) obtained by the production method of the present embodiment and an isocyanate component.

The amount of odor is reduced using the compound obtained by the production method of the compound (1) according to the above embodiment. In addition, by the method of producing a spectacle lens according to the present embodiment, a polythiourethane resin having a high refractive index and a colorless and transparent appearance is obtained.

The method of producing a spectacle lens according to the present embodiment may include annealing the cured resin.

The polymerization is preferably a cast polymerization method. For example, the lens substrate can be obtained by injecting a polymerizable composition into a mold in which a glass or metal mold and a tape or a gasket are combined and performing polymerization.

Polymerization conditions can be appropriately set depending on the polymerizable composition. The polymerization start temperature is preferably 0°C or higher, more preferably 10°C or higher, and preferably 50°C or lower, and more preferably 40°C or lower. Preferably, the temperature is raised from the polymerization start temperature and curing by heating is then performed. For example, the maximum heating temperature is generally 110°C or higher and 130°C or lower.

After the polymerization is completed, the lens substrate may be released, and the annealing treatment may be performed. The temperature during the annealing treatment is preferably 100 to 150°C.

As described above, the present disclosure discloses the following embodiments.

<1> A method of producing pentaerythritol mercaptocarboxylate, including subjecting a mercaptocarboxylic acid and pentaerythritol to a dehydration reaction under solvent-free conditions.

<2> The method of producing pentaerythritol mercaptocarboxylate according to <1>, wherein the mercaptocarboxylic acid has 2 to 4 carbon atoms.

<3> The method of producing pentaerythritol mercaptocarboxylate according to <1> or <2>, wherein the mercaptocarboxylic acid is 2-mercaptoacetic acid or 3-mercaptopropionic acid.

<4> The method of producing pentaerythritol mercaptocarboxylate according to any one of <1> to <3>, wherein the dehydration reaction is performed in the presence of a sulfonic acid compound.

<5> The method of producing pentaerythritol mercaptocarboxylate according to <4>, wherein the amount of the sulfonic acid compound is 0.1 to 10 mass% based on a total amount of the mercaptocarboxylic acid and the pentaerythritol.

<6> The method of producing pentaerythritol mercaptocarboxylate according to <4> or <5>, wherein the sulfonic acid compound is an alkylsulfonic acid.

<7> The method of producing pentaerythritol mercaptocarboxylate according to any one of <1> to <6>, wherein the dehydration reaction is performed under a reduced pressure while removing water from the system.

<8> The method of producing pentaerythritol mercaptocarboxylate according to any one of <1> to <7>, wherein the dehydration reaction is performed at a temperature of 90 to 98°C.

<9> The method of producing pentaerythritol mercaptocarboxylate according to any one of <1> to <8>, wherein the pentaerythritol mercaptocarboxylate has a Hazen color scale value (APHA) of 15 or less.

<10> The method of producing pentaerythritol mercaptocarboxylate according to any one of <1> to <9>, wherein the pentaerythritol contains more than 1.0 mass% of sodium.

<11> The method of producing pentaerythritol mercaptocarboxylate according to any one of <1> to <10>, including washing the pentaerythritol mercaptocarboxylate obtained by the dehydration reaction.

<12> A polymerizable composition including the pentaerythritol mercaptocarboxylate obtained by the production method according to any one of <1> to <11> and a polyiso(thio)cyanate.

<13> A resin which is a cured product of the polymerizable composition according to <12>.

<14> An optical material including the resin according to <13>.

<15> A spectacle lens including the resin according to <13>.

[Examples]

Hereinafter, the present embodiment will be described in more detail with reference to examples and comparative examples. Here, the present invention is not limited to the following examples.

[Measurement method]

<Hazen color scale value (APHA)>

The Hazen color scale value (APHA) of the obtained pentaerythritol mercaptocarboxylate was measured by the method according to JIS K0071-1: 2017. A color standard solution "1000" (product name, commercially available from FUJIFILM Wako Pure Chemical Corporation) was diluted with pure water to prepare solutions of APHA, 0, 5, 10, 15, 20, and 25, and a calibration curve was created. The obtained pentaerythritol mercaptocarboxylate was filled into a quartz cell with an optical path length of 50 mm, and the APHA value was measured using a Spectral Haze Meter "COH7700" (product name, commercially available from Nippon Denshoku Industries Co., Ltd.).

<Refractive index>

The refractive index of pentaerythritol mercaptocarboxylate was measured using a refractometer "RA-600" (product name, commercially available from Kyoto Electronics Manufacturing Co., Ltd.) at 25°C.

<SH value (SHV)>

The SH value (hereinafter referred to as "SHV") was determined by iodometric titration.

About 0.2 g of pentaerythritol mercaptocarboxylate was accurately weighed out into a 300 mL conical beaker. 50 mL of a mixed solvent of methanol:chloroform=2:5 was measured with a graduated cylinder, added to the conical beaker, and stirred and dissolved with a stirrer for 20 minutes.

For the blank measurement, only the solvent was put into a conical beaker and placed in cold water at 10±5°C, and while stirring with a stirrer at 300 rpm, iodometric titration was performed using a burette containing 0.05 mol/L of iodine aqueous solution. The point at which the solution turned yellow was set as the end point, the amount added dropwise was recorded, and this was used as the blank.

Next, the sample was dissolved in the solvent, and iodometric titration was performed using the same operation as above. The end point was confirmed, and the amount added dropwise was recorded. The SH value was obtained by inputting the amount added dropwise into the SH-value calculation formula.

$$SHV(g/eq) = (S \times 1000) / \{(A-B) \times f \times 0.1\}$$

A: titration amount of 0.05 mol/L iodine solution
B: blank value of 0.05 mol/L iodine solution
f: factor of 0.05 mol/L iodine solution
S: sample amount (g)

<Example 1>

37.3 parts by mass of pentaerythritol (0.274 mol), 120.0 parts by mass of 3-mercaptopropionic acid (1.131 mol) and 0.835 parts by mass of methanesulfonic acid were put into a 1,000 mL flask, and the flask was immersed in an oil bath whose temperature was controlled to about 100°C and stirring and reaction were performed while degassing using a vacuum pump for 3 hours (an internal temperature of 95°C).

After the reaction was completed, the sample was washed with 200 mL of water three times, and water was removed using a vacuum pump. The refractive index $n_d(25°C)$ of the synthesized pentaerythritol mercaptocarboxylate was 1.528, and the APHA value was 5.

53.15 parts by mass of 1,3-bis(isocyanatomethyl)cyclohexane (hereinafter referred to as "HXDI"), 0.22 parts by mass of acidic phosphate ester "JP506H" (product name, commercially available from Johoku Chemical Co., Ltd.), 0.072 parts by mass of dimethyltin dichloride (DMTDCI), and 0.12 parts by mass of a UV absorbing agent "SEESORB707" (product name, commercially available from Shipro Kasei Kaisha, Ltd.) were mixed and dissolved, 66.85 parts by mass of the pentaerythritol mercaptocarboxylate described above was added, the mixture was stirred and degassed for 30 minutes to make it homogeneous, the mixture was filtered through a 10-micron PTFE filter, and 50 g of the mixture was poured into a glass sample bottle and sealed. The mixture was polymerized by heating from 20°C to 120°C for 24 hours and cooled to near room temperature, and the resin cylinder in the sample bottle was removed. The obtained resin cylinder was evaluated as described above, and the results are shown in Table 1.

<Examples 2 and 3>

Pentaerythritol mercaptocarboxylates and resin cylinders were obtained in the same manner as in Example 1 except that raw materials were changed as shown in Table 1. The obtained resin cylinder was evaluated as described above, and the results are shown in Table 1.

<Example 4>

Pentaerythritol mercaptocarboxylate and a resin cylinder were obtained in the same manner as in Example 1 except that, as pentaerythritol, a mixture containing 37.3 parts by mass of pentaerythritol and 1.10 parts by mass of NaCl was used. The obtained resin cylinder was evaluated as described above, and the results are shown in Table 1.

<Comparative Example 1>

In the same manner as in Example 1, 37.3 parts by mass of pentaerythritol (0.274 mol), 120.0 parts by mass of 3-mercaptopropionic acid (1.131 mol), 1.04 parts by mass of p-toluenesulfonic acid/monohydrate, and 53.2 parts by mass of toluene were put into a 1,000 mL flask. The reaction was performed at about 100°C for 7 hours while heating under reflux and removing water from the system, and cooling was then performed. The toluene solution was washed with 200 mL of water three times, and toluene and water were distilled off under heating and a reduced pressure. The obtained resin cylinder was evaluated as described above, and the results are shown in Table 1. The refractive index $n_d$ (25°C) of the synthesized pentaerythritol mercaptopropionate was 1.53, and the APHA value was 8.

[Table 1]

| Table 1 | | Example 1 | Example 1 | Example 1 | Example 1 | Comparative Example 1 |
|---|---|---|---|---|---|---|
| Raw materials (parts by mass) | Pentaerythritol | 37.3 | 37.3 | 37.3 | 37.3 | 37.3 |
| | 3-mercaptopropionic acid | 120.0 | 120.0 | 116.3 | 120.0 | 120.0 |
| | methanesulfonic acid | 0.835 | 0 | 0.836 | 0.835 | 0.835 |
| | toluenesulfonic acid | 0 | 0.835 | 0 | 0 | 0 |
| | NaCl | 0 | 0 | 0 | 1.10 | 1.10 |
| Solvent | | solvent-free | solvent-free | solvent-free | solvent-free | toluene |
| Reaction temperature (°C) | | 95 | 100 | 100 | 95 | 100 |
| Monomer evaluation | Refractive index (25°C) | 1.53 | 1.52 | 1.53 | 1.52 | 1.53 |
| | SHV | 128.3 | 130.1 | 128.6 | 128.1 | 128.3 |
| | APHA | 5 | 8 | 7 | 5 | 8 |

On the basis of the above results, it can be understood that there is provided a method of producing pentaerythritol mercaptocarboxylate with less purification load and less coloration according to a dehydration reaction in solvent-free conditions.

## Claims

1.  A method of producing pentaerythritol mercaptocarboxylate, comprising
    subjecting a mercaptocarboxylic acid and pentaerythritol to a dehydration reaction under solvent-free conditions.

2.  The method of producing pentaerythritol mercaptocarboxylate according to claim 1,
    wherein the mercaptocarboxylic acid has 2 to 4 carbon atoms.

3.  The method of producing pentaerythritol mercaptocarboxylate according to claim 1,
    wherein the mercaptocarboxylic acid is 2-mercaptoacetic acid or 3-mercaptopropionic acid.

4.  The method of producing pentaerythritol mercaptocarboxylate according to claim 1,
    wherein the dehydration reaction is performed in the presence of a sulfonic acid compound.

5.  The method of producing pentaerythritol mercaptocarboxylate according to claim 4,
    wherein the amount of the sulfonic acid compound is 0.1 to 10 mass% based on a total amount of the mercaptocarboxylic acid and the pentaerythritol.

6.  The method of producing pentaerythritol mercaptocarboxylate according to claim 4,
    wherein the sulfonic acid compound is an alkylsulfonic acid.

7.  The method of producing pentaerythritol mercaptocarboxylate according to claim 1,
    wherein the dehydration reaction is performed under a reduced pressure while removing water from the system.

8.  The method of producing pentaerythritol mercaptocarboxylate according to claim 1,
    wherein the dehydration reaction is performed at a temperature of 90 to 98°C.

9. The method of producing pentaerythritol mercaptocarboxylate according to claim 1,
   wherein the pentaerythritol mercaptocarboxylate has a Hazen color scale value (APHA) of 15 or less.

10. The method of producing pentaerythritol mercaptocarboxylate according to claim 1,
    wherein the pentaerythritol contains more than 1.0 mass% of sodium.

11. The method of producing pentaerythritol mercaptocarboxylate according to claim 1, comprising
    washing the pentaerythritol mercaptocarboxylate obtained by the dehydration reaction.

12. A polymerizable composition comprising the pentaerythritol mercaptocarboxylate obtained by the production method
    according to any one of claims 1 to 11 and a polyiso(thio)cyanate.

13. A resin which is a cured product of the polymerizable composition according to claim 12.

14. An optical material comprising the resin according to claim 13.

15. A spectacle lens comprising the resin according to claim 13.

## INTERNATIONAL SEARCH REPORT

| | International application No. |
|---|---|
| | **PCT/JP2023/013187** |

**A.    CLASSIFICATION OF SUBJECT MATTER**

*C07C 319/12*(2006.01)i; *C07C 321/04*(2006.01)i; *C07B 61/00*(2006.01)i; *C08G 18/38*(2006.01)i; *G02B 1/04*(2006.01)i
FI:    C07C319/12; C08G18/38 076; C07C321/04; G02B1/04; C07B61/00 300

According to International Patent Classification (IPC) or to both national classification and IPC

**B.    FIELDS SEARCHED**

Minimum documentation searched (classification system followed by classification symbols)

C07C319/12; C07C321/04; C07B61/00; C08G18/38; G02B1/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN)

**C.    DOCUMENTS CONSIDERED TO BE RELEVANT**

| Category* | Citation of document, with indication, where appropriate, of the relevant passages | Relevant to claim No. |
|---|---|---|
| X | JP 11-080117 A (ASAHI DENKA KOGYO KK) 26 March 1999 (1999-03-26) | 1-3, 7-9, 11 |
| | claims, paragraphs [0015], [0016] | |
| Y | claims, paragraphs [0015], [0016] | 4-6, 10, 12-15 |
| Y | JP 2011-126822 A (SHOWA DENKO KK) 30 June 2011 (2011-06-30) | 4-6 |
| | claims, paragraphs [0049]-[0051] | |
| Y | JP 57-011959 A (ASAHI DENKA KOGYO KK) 21 January 1982 (1982-01-21) | 4-6 |
| | page 3, lower right column, lines 6-20 | |
| Y | WO 2007/052329 A1 (MITSUI CHEMICALS, INC.) 10 May 2007 (2007-05-10) | 10 |
| | paragraphs [0061]-[0064] | |
| Y | WO 2016/208707 A1 (MITSUI CHEMICALS, INC.) 29 December 2016 (2016-12-29) | 12-15 |
| | claims, paragraphs [0056]-[0067] | |
| X | JP 2011-084479 A (SHOWA DENKO KK) 28 April 2011 (2011-04-28) | 1-2, 4-5, 7-8, 11 |
| | claims, paragraphs [0191]-[0195] | |
| Y | claims, paragraphs [0191]-[0195] | 6, 10, 12-15 |

☐ Further documents are listed in the continuation of Box C.       ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **12 May 2023** | **30 May 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

| Patent document cited in search report | | | Publication date (day/month/year) | Patent family member(s) | Publication date (day/month/year) |
|---|---|---|---|---|---|
| JP | 11-080117 | A | 26 March 1999 | (Family: none) | |
| JP | 2011-126822 | A | 30 June 2011 | (Family: none) | |
| JP | 57-011959 | A | 21 January 1982 | (Family: none) | |
| WO | 2007/052329 | A1 | 10 May 2007 | US 2009/0270583 A1 paragraphs [0076]-[0079] | |
| WO | 2016/208707 | A1 | 29 December 2016 | US 2018/0186733 A1 claims, paragraphs [0119]-[0132] | |
| JP | 2011-084479 | A | 28 April 2011 | (Family: none) | |

Form PCT/ISA/210 (patent family annex) (January 2015)

**EP 4 506 335 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004 A
- JP 002820 A
- WO 2016208707 A